# EUROPEAN PATENT SPECIFICATION

(11) **EP 2 430 152 B1**
(45) Date of publication and mention of the grant of the patent: **24.08.2016**
(21) Application number: 11791415.0
(22) Date of filing: 15.07.2011
(51) Int. Cl.: C12N 1/21, C12N 9/12, C12P 13/08

(54) **MICROORGANISM WITH ENHANCED L-LYSINE PRODUCTIVITY AND METHOD FOR PRODUCING L-LYSINE USING THE SAME**
MIKROORGANISMUS MIT VERSTÄRKTER L-LYSIN-PRODUKTIVITÄT UND VERFAHREN ZUR L-LYSIN-HERSTELLUNG DAMIT
MICRO-ORGANISME AYANT UNE PRODUCTIVITÉ DE L-LYSINE AMÉLIORÉE ET PROCÉDÉ DE PRODUCTION DE L-LYSINE L'UTILISANT

(30) Priority: 15.07.2010 KR 20100068618; 01.07.2011 KR 20110065694
(43) Date of publication of application: 21.03.2012
(73) Proprietor: CJ CheilJedang Corporation, Seoul, 100-400 (KR)
(72) Inventor: LIM, Sang Jo, Incheon 402-040 (KR); MOON, Jun Ok, Seoul 158-094 (KR); KIM, Hyung Joon, Seoul 152-773 (KR); JANG, Jae Woo, Suwon-si Gyeonggi-do 442-070 (KR); CHO, Jae Yong, Wonju-si Gangwon-do 220-976 (KR)
(74) Representative: Gevers & Orès
(86) International application number: PCT/KR2011/005252
(87) International publication number: WO 2012/008810

(56) References cited:
- WO-A1-2008/082180
- WO-A1-2008/082181
- WO-A2-02/081440
- WO-A2-2005/017099
- WO-A2-2012/008809
- KR-A- 20070 056 805
- KR-A- 20070 056 807
- US-B2- 7 332 310
- M. LETEK ET AL: "Characterization and Use of Catabolite-Repressed Promoters from Gluconate Genes in Corynebacterium glutamicum", JOURNAL OF BACTERIOLOGY, vol. 188, no. 2, 15 January 2006 (2006-01-15), pages 409-423, XP055064370, ISSN: 0021-9193, DOI: 10.1128/JB.188.2.409-423.2006
- A PORCO ET AL: "Gluconate as suitable potential reduction supplier in Corynebacterium glutamicum: cloning and expression of gntP and gntK in Escherichia coli", BIOL RES, vol. 41, 28 March 2008 (2008-03-28), pages 349-358, XP055064371,
- JULIA FRUNZKE ET AL: "Co-ordinated regulation of gluconate catabolism and glucose uptake in Corynebacterium glutamicum by two functionally equivalent transcriptional regulators, GntR1 and GntR2", MOLECULAR MICROBIOLOGY, vol. 67, no. 2, 6 November 2007 (2007-11-06), pages 305-322, XP055064372, ISSN: 0950-382X, DOI: 10.1111/j.1365-2958.2007.06020.x
- GUI-HYE HWANG ET AL: "Implication of gluconate kinase activity in-ornithine biosynthesis in", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 39, no. 12, 18 September 2012 (2012-09-18), pages 1869-1874, XP035140097, ISSN: 1476-5535, DOI: 10.1007/S10295-012-1197-7
- DATABASE UNIPROT [Online] 01 October 2002 'SubName: Full=Gluconate kinase; SubName: Full=PUTATIVE GLUCONOKINASE; EC=2.7.1.12;' Retrieved from EBI, accession no. UNIPROT:Q8NMT0 Database accession no. Q8NMT0
- DATABASE UNIPROT [Online] 01 October 2002 'SubName: Full=PUTATIVE GLUCONATE KINASE; EC=2.7.1.12; SubName: Full=Sugar (Pentulose and hexulose) kinases; EC=2.7.1.12;' Retrieved from EBI, accession no. UNIPROT:Q8NLD4 Database accession no. Q8NLD4

## Description

### Technical Field

The present invention relates to a microorganism with high L-lysine productivity and a method for producing L-lysine using the same.

### Background Art

L-Lysine is synthesized from oxaloacetic acid via the lysine biosynthetic pathway. The enzymes important to the biosynthesis of L-lysine are aspartate semialdehyde dehydrogenase, dihydrodipicolinate reductase and diaminopimelate dehydrogenase, which are respectively encoded by the genes asd, dapB and ddh, and which are NADPH-dependent reductases that mediate parts of the lysine biosynthetic pathway. In this pathway, the production of one molecule of L-lysine directly requires the consumption of three molecules of NADPH by the enzymes and one molecule of NADPH indirectly. Direct correlation between the regeneration of NADPH and the biosynthesis of L-lysine in the *Corynebacterium glutamicum* cell was previously reported (Wittmann and Heinzle, Microbiol 68:5843-5849, 2002; Marx et al., J Biotechnol 104:185-197, 2003; Ohnishi et al., Microbiol Lett 242:265-274, 2005).

In Corynebacterium, the main supply routes of NADPH are the TCA cycle and the pentose phosphate pathway. It is preferred that the reducing power necessary for the production of L-lysine be supplied via oxidation pathway of the pentose phosphate pathway. The pentose phosphate pathway is economically more beneficial in terms of carbon metabolism yield because the operation of two cycles of the pentose phosphate pathway releases one CO₂ molecule with the concomitant production of two NADPH molecules whereas two CO₂ molecules are released per one TCA cycle, with the concomitant production of one NADPH molecule. Thus, a higher yield of L-lysine requires a greater supply of NADPH, resulting in a higher dependence on the pentose phosphate pathway.

Glucose-6-phosphate dehydrogenase (G6PDH) and 6-phosphogluconate dehydrogenase (6PGD), both involved in the pentose phosphate pathway, were reported to be associated with the production of L-lysine (Marx et al., Biotechnol Bioeng 56:168-180, 1997; Wittmann and Heinzle, Microbiol 68:5843-5849, 2002).

In L-amino acid-producing Corynebacterium strains, when genes encoding enzymes responsible for the production of NADPH in the pentose phosphate pathway are enhanced or when the enzymes are mutated, L-amino acid productivity is reported to be increased. For example, J. Becker et al., reported that the provision of a stronger promoter for the zwf gene encoding G6PDH in L-lysine-producing C. *glutamicum* brought about a significant increase in lysine production (J. Becker et al., J Biotechnol 132: 99-109, 2007). European Patent No. 1302537 discloses a method for producing L-amino acids by culturing L-amino acid-producing corynebacteria into a gene encoding a mutant of 6PGD which has been introduced.

A Corynebacterium sp. microorganism in which the NADPH biosynthesis in the pentose phosphate pathway was increased by attenuating the endogenous gluconate kinase (GntK) activity of Corynebacterium, however, has not been disclosed in previous reports.

### Disclosure of Invention

### Technical Problem

Leading to the present invention, intensive and thorough research, conducted by the present inventors, aimed at finding a method for production of L-lysine at high efficiency and yield, resulted in the finding that an increase in the production of NADPH by genetic modification in the oxidative pentose phosphate pathway can increase yield of L-lysine.

### Solution to Problem

To solve said technical problem, the Inventors have developed an L-lysine-producing microorganism that has gluconate kinase (GntK) activity weakened in comparison with the endogenous activity and
a method for producing the L-lysine-producing microorganism, comprising: constructing a polynucleotide fragment encoding gluconate kinase (GntK) having a weakened activity by wholly or partially mutating said polynucleotide; inserting the polynucleotide fragment into a vector to afford a recombinant vector, said vector being capable of homologous recombination with a chromosome in a host cell; introducing the recombinant vector into a host cell capable of producing L-lysine to form homologous recombinants; and selecting a strain having GntK activity weakened in comparison to the endogenous activity thereof, from the homologous recombinants.

It is therefore an object of the present invention to provide a method for producing L-lysine, comprising: culturing the microorganism of the present invention having the activity of a gluconate kinase (GntK) weakened in comparison with the endogenous activity thereof in the wild-type *Corynebacterium spp.,* wherein the GntK has the amino acid sequence SEQ ID NO: 2, to obtain a cell culture; and harvesting L-lysine from the cell culture or the microorganism, wherein the GntK activity is weakened by whole or partial deletion, partial substitution or insertion:
- on a chromosomal gene encoding gluconate kinase or
- on a regulatory element for a chromosomal gene encoding gluconate kinase.

It is another object of the invention to use of the microorganism of the present invention that has gluconate kinase (GntK) activity weakened in comparison with the endogenous activity for producing L-lysine, wherein the GntK activity is weakened by whole or partial deletion, partial substitution or insertion:
- on a chromosomal gene encoding gluconate kinase or
- on a regulatory element for a chromosomal gene encoding gluconate kinase.

### Advantageous Effects of Invention

The present invention can produce L-amino acids in need of NADPH for their biosynthesis at high efficiency and yield, especially L-lysine, via increasing the intracellular level of NADPH in Corynebacterium strains.

### Brief Description of Drawings

The above and other objects, features and other advantages of the present invention will be more clearly understood from the following detailed description taken in conjunction with the accompanying drawings, in which:
FIG. 1 is a schematic diagram illustrating the pentose phosphate pathway in *Corynebacterium glutamicum*;
FIG. 2 is a schematic diagram illustrating the pDZ-ΔNCgl2399 vector for marker-free deletion of the chromosomal NCgl2399 gene of Corynebacterium; and
FIG. 3 is a schematic diagram illustrating the pDZ-ΔNCgl2905 vector for marker-free deletion of the chromosomal NCgl2905 gene of Corynebacterium.

### Best Mode for Carrying out the Invention

In accordance with an aspect thereof, the present invention provides a method for producing L-lysine, comprising the steps of: 1) culturing an L-lysine-producing microorganism belonging to a *Corynebacterium spp.,* having the activity of a gluconate kinase (GntK) weakened in comparison with the endogenous activity thereof in the wild-type *Corynebacterium spp.,* wherein the GntK has the amino acid sequence SEQ ID NO: 2, to obtain a cell culture; and 2) harvesting L-lysine from the cell culture or the microorganism,
wherein the GntK activity is weakened by whole or partial deletion, partial substitution or insertion:
- on a chromosomal gene encoding gluconate kinase or
- on a regulatory element for a chromosomal gene encoding gluconate kinase.

In accordance with another aspect thereof, the invention provides the use of a microorganism belonging to a *Corynebacterium, spp.,* having the activity of a gluconate kinase (GntK) weakened in comparison with the endogenous activity thereof in the wild-type *Corynebacterium, spp.,* wherein the GntK has the amino acid sequence SEQ ID NO: 2, for producing L-lysine, wherein the GntK activity is weakened by whole or partial deletion, partial substitution or insertion:
- on a chromosomal gene encoding gluconate kinase or
- on a regulatory element for a chromosomal gene encoding gluconate kinase.
L-lysine is a basic α-amino acid with the chemical formula of NH₂(CH₂)₄CH(NH₂ )COOH. It is an essential amino acid, which means that the human body cannot synthesize it. L-lysine is synthesized from oxaloacetic acid via the lysine biosynthesis pathway a part of which is catalyzed by NADPH-dependent reductases. The biosynthesis of one L-lysine molecule via this pathway requires the direct consumption of three NADPH molecules by enzymes, with the indirect use of one NADPH molecule.

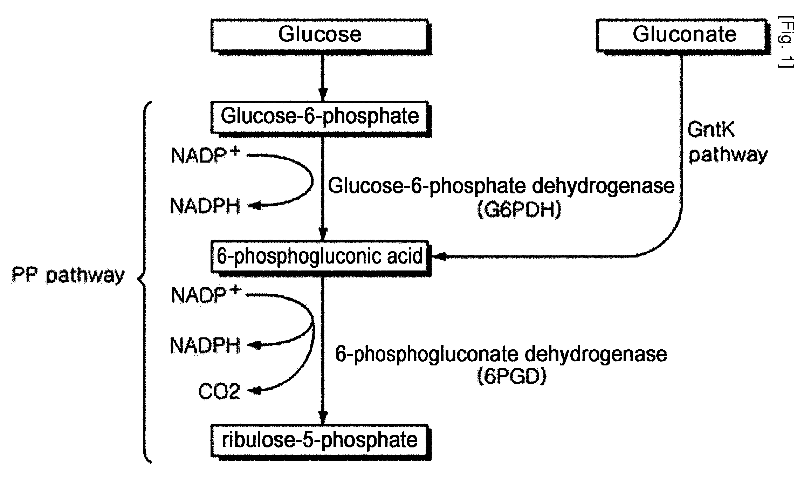

The term "gluconate kinase (GntK)," as used herein, refers to an enzyme that is concerned in the GntK pathway which produces 6-phosphogluconic acid, an intermediate in oxidative process of the pentose phosphate pathway in microorganisms. Microorganisms of Corynebacterium spp. operate both pathways, that is, the G6PDH and GntK pathways, to synthesize 6-phosphogluconic acid, an intermediate of the pentose phosphate pathway (FIG. 1), which means that microorganisms of Corynebacterium spp. may partially degrade glucose via the GntK pathway. On the presumption that the blockage of the GntK pathway may interrupt the provision of carbon to 6-phosphogluconic acid and convert carbon flux from 6-phosphogluconic acid to the oxidation process of the pentose phosphate pathway, the present inventors suggest that the blockage of the GntK pathway gives rise to a specific increase in the activity of 6-phosphogluconate dehydrogenase (6PGD), thus increasing the regeneration of NADPH. In this context, the activity of the enzyme which is presumed to act as gluconate kinase (GntK) in microorganisms is weakened.

In the method and in the use of the invention, the gluconate kinase (GntK) can further comprises an amino acid sequence of SEQ ID NO: 1.

Preferably, the enzyme having gluconate kinase (GntK) activity represented by the amino acid sequence of SEQ ID NO: 1 may be NCgl2399 and the enzyme having gluconate kinase (GntK) activity represented by the amino acid sequence of SEQ ID NO: 2 may be NCgl12905.

As used herein, the term "endogenous activity" is intended to mean the activity of an enzyme in a native microorganism, and particularly when used in combination with GntK, refers to the activity of GntK that a native microorganism exhibits.

The term "weakness of endogenous activity" is achieved on a chromosome containing a polynucleotide encoding an endogenous protein in a microorganism, by a method selected from the group consisting of a whole or partial deletion of the polynucleotide sequence, partial substitution of the polynucleotide sequence, or at least one base pair insertion into the polynucleotide sequence. In addition, endogenous activity can be weakened by mutating a regulatory element for the polynucleotide sequence, where the mutation is achieved by whole or partial deletion, substitution, or insertion of the regulatory element. The regulatory element which is mutated may be on the upstream or downstream of the polynucleotide sequence in a chromosome, preferably, the regulatory element may be a promoter, enhancer *et, al,* but the present invention is not limited thereto. The mutation of the polynucleotide may be achieved using a well-known method, such as a homologous recombination.

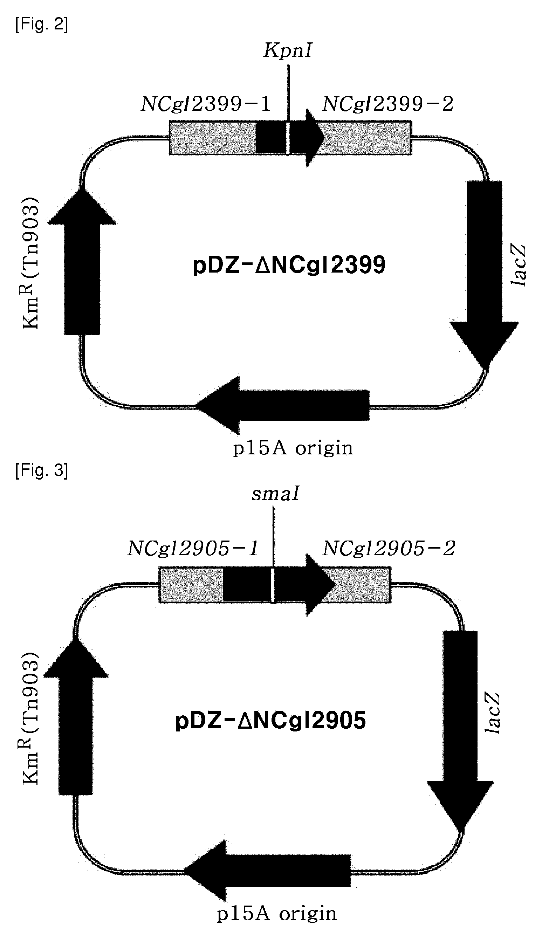

The term "the activity of a gluconate kinase (GntK) weakened in comparison to the endogenous activity," as used herein, is intended to mean that GntK activity is down-regulated by genetic mutation such as disruption and thus becomes lower than the endogenous activity of the enzyme in a native microorganism. In an embodiment of the present invention, the present invention provides microorganisms of Corynebacterium spp. with enhanced L-lysine productivity by disrupting GntK activity via introducing a recombinant vector represented by the cleavage map in Fig 2 or 3.

When there are two or more chromosomal genes coding proteins having gluconate kinase activity and having different amino acid sequences to each other, the endogenous GntK activity can be weakened by whole or partial deletion, substitution or insertion on at least one of the chromosomal genes. And endogenous GntK activity can be weakened by whole or partial deletion, partial substitution or insertion on a regulatory element for at least one of the chromosomal genes.

Preferably, a mutation by deletion, substitution or insertion of nucleotides may be allowed to occur on a gene encoding NCgl2399 or NCgl2905, or either one or both of the genes having nucleotide sequences of SEQ ID NOS: 3 and 4 which code respectively for NCgl2399 and NCgl2905, or on regulatory elements thereof, so that GntK does not function normally.

In the present invention, a microorganism with gluconate kinase activity weakened in comparison with the endogenous activity thereof can be prepared by introducing a recombinant vector carrying a part of the gene encoding gluconate kinase thereinto to delete or mutate the endogenous gluconate kinase gene thereof. The insertion of the partial gene into the chromosome may be achieved using a well-known method, such as a homologous recombination.

The term "recombinant vector," as used herein, is intended to refer to a vector that cannot be replicated separately from the chromosome of a host cell and typically means a genetic construction comprising essential elements which allow the homologous recombination of a foreign gene with the chromosome. The recombinant vector may carry an antibiotic-resistance gene and a selection gene such as levansucrase (*sacB*) gene. Preferably, the recombinant vectors represented by the cleavage maps of FIGS. 2 and 3 may be employed in the present invention.

As long as L-lysine can be produced, any strain may be used without limitation as a host cell into which the recombinant vector is introduced. Preferred are the microorganisms of Corynebacterium spp. or Brevibacterium spp. Examples of host cells useful in the present invention include *C*. *glutamicum* ATCC13032, C. *ther-moaminogenes* FERM BP-1539, *Brevibacterium flavum* ATCC 14067, and *Bre-vibacterium lactofermentum* ATCC 13869, and L-amino acid-producing mutants or stains derived therefrom, such as *C*. *glutamicum* KFCC10881, *C. glutamicum* KFCC11001 and *C*. *glutamicum* KCCM10770P. Preferred is *C*. *glutamicum* KFCC10881. In an embodiment of the present invention, *C*. *glutamicum* KFCC10881 was employed, but the present invention is not limited thereto.

In an embodiment of the present invention, the pDZ vector (disclosed in Korean Patent No. 0924065), which can perform the marker-free deletion of a target gene in *C*. *glutamicum* without replication, was utilized to delete the genes. That is, a recombinant vector carrying a part of the gene encoding NCgl2399 or NCgl2905 was constructed, transformed into an L-lysine-producing Corynebacterium strain, and allowed to perform homologous recombination with the genome to prepare L-lysine-producing Corynebacterium strain having weakened gluconate kinase activity.

In the present invention, a part of a gene encoding NCgl2399 was inserted into a pDZ vector to construct a recombinant vector, called pDZ-ANCgl2399, which was then transformed into a Corynebacterium strain. The resulting recombinant Corynebacterium strain in which the chromosomal gene encoding NCgl2399 was disrupted was named KFCC10881-ΔNCgl2399. Separately, a recombinant pDZ vector carrying a part of a gene encoding NCgl2905, named pDZ-ANCgl2905, was transformed into a Corynebacterium strain. The resulting recombinant strain in which the chromosomal gene encoding NCgl2905 was disrupted was named KFCC10881-ΔNCgl2905. Further, KFCC10881-ΔNCgl2399 was transformed with the recombinant plasmid pDZ-ΔNCgl2905 to provide a mutant strain which was defective in both NCgl2399 and NCgl2905 genes. The strain was named *C. glutamicum* CA01-0892 and deposited with the Korean Culture Center of Microorganisms on June 24,2010, under accession No. KCCM 11085P.

Further, in one embodiment of the present invention, the recombinant C. glutamicum strains were evaluated for intracellular gluconate kinase activity and NADPH level. The intracellular GntK activity of the strain which is defective in the NCgl2399 gene and the strain which is defective in both the NCgl2399 and NCgl2905 gene were observed to have been decreased compared to the parent strain. In addition, intracellular NADPH level and 6PGD activity were observed to have been increased compared to the parent strain (Table 2). Consequentially, L-lysine production yield of the prepared strains were observed to have been increased compared to the parent strain(Table 3).

These results demonstrate that the attenuation of gluconate kinase activity below the endogenous activity increases 6PGD activity, which has a positive effect on the generation of NADPH, thus resulting in the production of L-lysine at high efficiency and yield. In addition, in the case where a protein having gluconate kinase activity is encoded by two or more different genes, higher L-lysine productivity can be obtained by deleting two or more of the genes. Therefore, the L-lysine-producing microorganisms of the present invention can be used for producing L-lysine at high efficiency and yield.

As used herein, the term "culturing" means allowing microorganisms to grow under artificially controlled conditions. Various methods well known in the art may be employed to culture the recombinants of the present invention. The cells may be cultured in a batch process or in a continuous process such as a fed-batch process or a repeated fed batch process, but the present invention is not limited thereto.

For use in the culturing, a medium must satisfy the requirement of the strain employed. Culture media suitable for use in culturing Corynebacteria strains are well known in the art (e.g., Manual of Methods for General Bacteriology, American Society for Bacteriology, Washington D.C., USA, 1981). Carbon sources of the culture media may be saccharides and carbohydrates such as glucose, sucrose, lactose, fructose, maltose, starch and cellulose; oils and lipids such as soybean oil, sunflower seed oil, peanut oil and coconut oil; fatty acids such as palmeatic acid, stearic acid, rinoleic acid;, alcohols such as glycerol and ethanol; and organic acids such as acetic acid. These materials may be used separately or in combination. Examples of a nitrogen source useful for culturing the recombonants include peptone, yeast extract, broth, malt extract, corn steep liquor, soybean meal and urea, or inorganic compounds such as ammonium sulfate, ammonium chloride, ammonium phosphate, ammonium carbonate and ammonium nitrate. These nitrogen sources may be used separately or in combination. Among phosphorus sources useful in the culture media are dipotassium hydrogen phosphate, potassium dihydrogen phosphate and corresponding sodium salts. In addition, culture media may contain metal salts essential to the growth of cells and may be supplemented with essential nutrients such as amino acids and vitamins. In addition, proper precursors may be added to the culture media. The nutrients or supplements may be added altogether once or in separation during cultivation.

The pH of the culture media may be adjusted with a basic compound such as sodium hydroxide, potassium hydroxide or ammonia or an acidic compound such as phosphoric acid or sulfuric acid. The generation of foam in the culture media may be restrained using an anti-foaming agent such as fatty acid polyglycol ester. The culture media may be kept under aerobic conditions by introducing oxygen or an oxygen-containing gas (e.g., air) thereinto. The culture temperature is typically between 20 and 45°C and preferably between 25 and 40°C. The culturing is continued until a maximal amount of L-amino acid is produced. In this regard, it may be accomplished within 10 to 160 hrs. After being produced, the L-lysine may be exported into the culture media or may remain within the cells.

The method and use of the invention for the production of L-lysine comprises harvesting lysine from the cells or the cell culture. L-lysine can be isolated from culture media or cells using a well-known method. Examples of the isolation method useful in the present invention include centrifugation, filtration, anionic exchange chromatography, crystallization and HPLC, but are not limited thereto.

### Mode for the Invention

A better understanding of the present invention may be obtained through the following examples which are set forth to illustrate, but are not to be construed as limiting the present invention.

### EXAMPLE 1: Preparation of Mutant Strains by Site-Specific Gene Disruption

For use in the preparation of the recombinant strains KFCC10881-ΔNCgl2399 and KFCC10881-ΔNCg12905, primers were designed. To begin with, sequences of NCgl2399(SEQ ID NO: 1) and NCgl2905(SEQ ID NO: 2) were obtained from the NIH GenBank. On the basis of these sequences, the primers 2399F1, 2399F2, 2399R1, 2399R2, 2905F1, 2905F2, 2905R1, and 2905R2 (Table 1) were synthesized which would be used in constructing inactivation fragments of NCgl2399 or NCgl2905. Nucleotide sequences of the primers used in the present invention, along with their SEQ ID NOs., are summarized in Table 1 wherein restriction sites are underlined.
Table 1

**[Table 1]**

| Primer | Nucleotide Sequence | SEQ ID No: |
|---|---|---|
| 2399F1 | gctctagaCCCCAGAACATGCTGACG(XbaI) | 5 |
| 2399R1 | ggggtaccCGCTGCTAGGGCTTTACC(KpnI) | 6 |
| 2399F2 | ggggtaccGGAACCGTCTTCGTCCACC(KpnI) | 7 |
| 2399R2 | gctctagaGTCACCGCTGGTACATCC(XbaI) | 8 |
| 2905F1 | gctctagaCATTGGAGCATCCGTAGC(XbaI) | 9 |
| 2905R1 | tcccccgggGCCTTCACCATCGACCAA(SmaI) | 10 |
| 2905F2 | tcccccgggGTTCCCGAACAGATCCCC(SmaI) | 11 |
| 2905R2 | gctctagaCGCTCTGACCTGCCTAAC(XbaI) | 12 |

Site-specific gene disruption was conducted with pDZ, which is incapable of replication in *C*. *glutamicum.* For use in preparing gene-disrupted mutant strains, pDZ derivatives carrying respective internally defective open reading frames of NCgl2399 and NCgl2905 were constructed. The pDZ derivatives are pDZ-ΔNCgl2399 (FIG. 2) and pDZ-ΔNCgl2905 (FIG. 3). pDZ-ΔNCgl2399 carries an *XbaI* end and an internal gene defective at a KpnI site of a 2,267 bp-long NCgl2399. The internal gene defective of NCgl2399 was generated using overlap extension PCR in the presence of 2399F1-2399R1 primers (SEQ ID NOS: 5 and 6) and 2399F2-2399R2 primers (SEQ ID NOS: 7 and 8), with the genomic DNA of C. *glutamicum* ATCC13032 serving as a template.

pDZ-ANCgl2905 carries a *XbaI* end and an internal gene defective at a SmaI site of a 2,819 bp-long NCgl2905. The internal gene defective of NCgl2905 was generated using overlap extension PCR in the presence of 2905F1-2905R1 primers (SEQ ID NOS: 9 and 10) and 2905F2-2905R2 primers (SEQ ID NOS: 11 and 12), with the genomic DNA of C. *glutamicum* ATCC13032 serving as a template.

The recombinant plasmids were transformed into wild-type Corynebacterium glutamicum using electroporation (van der Rest et al., Appl Microbiol Biotechnol 52:541-545, 1999) and incorporated into the chromosome by primary recombination (crossover). Then, the plasmids were excised from the chromosomes by secondary recombination (crossover) on an agar plate containing 10% sucrose.

Using gene-specific primer pairs 2399F1-2399R2 (SEQ ID NOS: 5 and 8) and 2905F1-2905R2 (SEQ ID NOS: 9 and 12), diagnostic PCR was performed on the genomic DNA of the C. *glutamicum* recombinants in which the secondary recombination was completed, confirming that the C. *glutamicum* recombinants had defective NCgl2399 and NCgl2905 genes, respectively. These recombinant strains were named KFCC10881-ΔNCgl2399 and KFCC10881-ΔNCgl2905, respectively. The open reading frame DNA sequences of NCgl2399 and NCgl2905 are referred to GenBank Accession No. NC_003450.

### EXAMPLE 2: Prepration of Mutant Strain Defective in Both NCgl2399 and NCgl2905 Genes

The recombinant strain KFCC10881-ΔNCgl2399 was transformed with the recombinant plasmid pDZ-ΔNCgl2905 using electroporation and treated in the same manner as illustrated above to give a novel recombinant strain that were defective in both NCgl2399 and NCgl2905 genes. This mutant strain was named *C*. *glutamicum* CA01-0892 and deposited with the Korean Culture Center of Microorganisms on June 24, 2010, under accession No. KCCM 11085P.

### EXAMPLE 3: Assay for Intracellular GntK and 6PGD Activity and NADPH Level

The L-lysine-producing strains *C. glutamicum* KFCC-10881-ΔNCgl2399, KFCC-10881-ΔNCgl2905 and CA01-0892 (KFCC-10881-ΔNCgL2399ΔNCgl2905), prepared in Examples 1 and 2, were assayed for intracellular gluconate kinase activity and the NADPH levels are as follows.

The parent strain *C*. *glutamicum* KFCC-10881 and the three recombinant strains were inoculated into respective 250 mL corner-baffle flasks containing 25 mL of the following complex medium and cultured at 30°C with shaking at 200 rpm. The cells in the exponential phase were harvested by centrifugation and suspended in 100 mM Tris/HCl buffer(pH 7.5). The cells were disrupted using glass beads, followed by centrifuging the cell lysate to obtain a supernatant.

### <Complex Medium (pH 7.0)>

Glucose 20 g, Peptone 10 g, Yeast Extract 5 g, Urea 1.5 g, KH₂PO₄ 4 g, K₂HPO₄ 8 g, MgSO₄ 7H₂O 0.5 g, Biotin 100 µg, Thiamine HCl 1000 µg, Calcium-pantothenate 2000 µg, Nicotine amide 2000 µg (in 1 liter of distilled water)

The supernatants were quantitatively analyzed for total protein contents by a Bradford method. 6PGD activity was determined using absorbance at 340 nm for NADPH (Frunzke et al., Mol Microbiol 67:305-322, 2008). GntK activity was measured by the coupled enzymatic assay for 6PGD (Frunzke et al., Mol Microbiol 67:305-322, 2008). NADPH levels were determined by an enzymatic cycling reaction with the aid of EnzyChrom™ NADP⁺/NADPH assay kit (BioAssay Systems, CA, USA).

As can be seen in Table 2, the intracellular GntK (EC:2.7.1.12) activity of KFCC-10881-ΔNCgl2399, which was defective in the NCgl2399 gene, was decreased by about 58.7% but there was a 2.4-fold increase in 6PGD activity, compared to the parent strain (KFCC10881). GntK activity of CA01-0892, defective in both NCgl2399 and NCgl2905 gene, was decreased by about 78.3%, but there was a 5.1-fold increase in 6PGD activity, compared to the parent strain (Table 2).

As a result of the enzymatic activity change, it was also observed that the intracellular NADPH level CA01-0892 was increased by up to 170%. These data were averages of measurements from at least three independent cultures, with standard deviations less than 10% in all cases. The results indicate that the attenuation of gluconate kinase activity below the endogenous activity increases 6PGD activity, which has a positive effect on the generation of NADPH.
Table 2

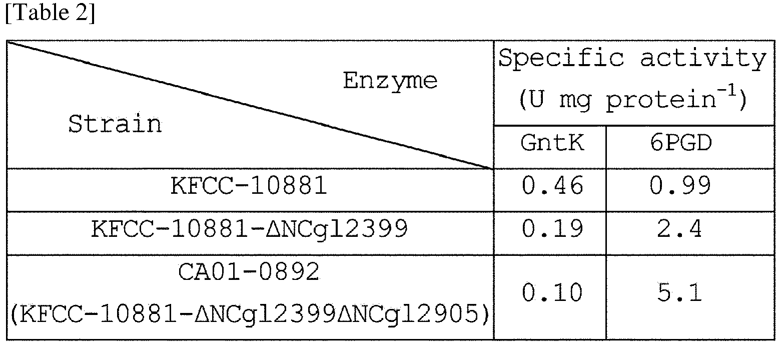

### EXAMPLE 4: Production of Lysine in Strains Defective in NCgl2399 and NCgl2905 Genes

To produce L-lysine, the L-lysine-producing strains *C. glutamicum* KFCC-10881-ΔNCgl2399, KFCC-10881-ΔNCgl2905 and CA01-0892 (KFCC-10881-ΔNCgl2399ΔNCgl2905), prepared in Examples 1 and 2, were cultured as follows.

The parent strain C. *glutamicum* KFCC-10881, and the three recombinant strains were inoculated into 250 mL-corner baffle flasks containing 25 mL of the following seed medium and cultured at 30°C for 20 hours with shaking at 200 rpm. Thereafter, 1 mL of each of the cultures were inoculated into a 250 mL-corner baffle flask containing 24 mL of the following production medium and cultured at 30°C for 120 hours with shaking at 200 rpm. The seed medium and the production medium comprise the following compositions.

### <Seed Medium (pH 7.0)>

Glucose 20 g, peptone 10 g, yeast extract 5 g, urea 1.5 g, KH₂PO₄ 4 g, K₂HPO₄ 8 g, MgSO₄ 7H₂O 0.5 g, biotin 100 µg, thiamine HCl 1000 µg, calcium-pantothenate 2000 µg, nicotinamide 2000 µg (in 1 liter of distilled water)

### <Production Medium (pH 7.0)>

glucose 100 g, (NH₄)₂SO₄ 40 g, soy protein 2.5 g, corn steep solids 5 g, urea 3 g, KH₂ PO₄ 1 g, MgSO₄ 7H₂O 0.5 g, biotin 100 µg, thiamine chloride 1000 µg, calcium-pantothenate 2000 µg, nicotinamide 3000 µg, and CaCO₃ 30 g (in 1 liter of distilled water).

After the completion of culture, HPLC analysis was performed to determine the amounts of the L-lysine produced by the strains. The concentrations of L-lysine in the cultures of *C*. *glutamicum* KFCC-10881, KFCC-10881-ΔNCgl2399, KFCC-10881-ΔNCgl2905 and CA01-0892(KFCC-10881-ΔNCgl2399ΔNCgl2905) are summarized in Table 3, below.
Table 3

**[Table 3]**

| Strain | Lysine (g/ℓ) | | |
|---|---|---|---|
| | Batch 1 | Batch 2 | Batch 3 |
| KFCC-10881 | 42.1 | 42.6 | 42.4 |
| KFCC-10881-ΔNCgl2399 | 44.9 | 45.5 | 45.2 |
| KFCC-10881-ΔNCgl2905 | 46.0 | 46.2 | 46.2 |
| CA01-0892 (KFCC-10881-ΔNCgl2399ΔNCgl2905) | 48.1 | 48.7 | 48.3 |

As can be seen in Table 3, the recombinant strains defective in the NCgl2399 or NCgl2905 gene, were found to increase in lysine productivity by about 5.9 % and 8.0 %, respectively, compared with the parent strain KFCC-10881. In addition, it was measured that an increase of about 13.1 % in lysine production was obtained with CA01-0892, defective in both NCgl2399 and NCgl2905 genes, in comparison to the parent strain (Table 3). These results demonstrate that the attenuation of gluconate kinase activity the endogenous activity increases 6PGD activity, which has a positive effect on the generation of NADPH, thus resulting in the production of L-lysine at high efficiency and yield.
## Claims

1. A method for producing L-lysine, comprising the steps of:
1) culturing an L-lysine-producing microorganism belonging to a *Corynebacterium spp.,* having the activity of a gluconate kinase (GntK) weakened in comparison with the endogenous activity thereof in the wild-type *Corynebacterium spp.,* wherein the GntK has the amino acid sequence SEQ ID NO: 2, to obtain a cell culture; and
2) harvesting L-lysine from the cell culture or the microorganism,
wherein the GntK activity is weakened by whole or partial deletion, partial substitution or insertion:
- on a chromosomal gene encoding gluconate kinase or
- on a regulatory element for a chromosomal gene encoding gluconate kinase.

2. The use of a microorganism belonging to a *Corynebacterium spp.,* having the activity of a gluconate kinase (GntK) weakened in comparison with the endogenous activity thereof in the wild-type *Corynebacterium spp.,* wherein the GntK has the amino acid sequence SEQ ID NO: 2, for producing L-lysine, wherein the GntK activity is weakened by whole or partial deletion, partial substitution or insertion:
- on a chromosomal gene encoding gluconate kinase or
- on a regulatory element for a chromosomal gene encoding gluconate kinase

3. The method of claim 1 or the use of claim 2, wherein the gluconate kinase (GntK) further comprises an amino acid sequence of SEQ ID NO: 1.

4. The method of claim 1. or the use of claim 2, wherein the L-lysine producing microorganism is .*Corynebacterium glutamicum* deposited under accession No. KCCM 11085P.

## Patentansprüche

1. Verfahren zur Herstellung von L-Lysin, das die folgenden Schritte umfasst:
1) Züchten eines L-Lysin herstellenden Mikroorganismus, der zu einem *Corynebacterium spp.* gehört, dessen Aktivität einer Gluconatkinase (GntK) im Vergleich zu seiner endogenen Aktivität im Wildtyp von *Corynebacterium spp.* geschwächt ist, wobei die GntK die Aminosäuresequenz SEQ ID NO: 2 aufweist, um eine Zellkultur zu erhalten; und
2) Ernten von L-Lysin von der Zellkultur oder dem Mikroorganismus, wobei die GntK-Aktivität durch gesamte oder partielle Deletion, partielle Substitution oder Insertion geschwächt wird:
- auf einem chromosomalen Gen, das Gluconatkinase kodiert oder
- auf einem Regulationselement für ein chromosomales Gen, das Gluconatkinase kodiert.

2. Verwendung eines Mikroorganismus, der zu einem *Corynebacterium spp.* gehört, dessen Aktivität einer Gluconatkinase (GntK) im Vergleich zu seiner endogenen Aktivität im Wildtyp von *Corynebacterium spp.* geschwächt ist, wobei die GntK die Aminosäuresequenz SEQ ID NO: 2 aufweist, zur Herstellung von L-Lysin, wobei die GntK-Aktivität durch gesamte oder partielle Deletion, partielle Substitution oder Insertion geschwächt wird:
- auf einem chromosomalen Gen, das Gluconatkinase kodiert oder
- auf einem Regulationselement für ein chromosomales Gen, das Gluconatkinase kodiert.

3. Verfahren nach Anspruch 1 oder Verwendung nach Anspruch 2, wobei die Gluconatkinase (GntK) ferner eine Aminosäuresequenz von der SEQ ID NO: 1 umfasst.

4. Verfahren nach Anspruch 1 oder Verwendung nach Anspruch 2, wobei der L-Lysin herstellende Mikroorganismus *Corynebacterium glutamicum* ist, der unter der Zugangsnummer KCCM 11085P angemeldet wurde.

## Revendications

1. Procédé de production de L-lysine, comprenant les étapes suivantes :
1) la culture d'un micro-organisme producteur de L-lysine appartenant à une *Corynebacterium spp.,* ayant l'activité d'une glucuronate kinase (GntK) affaiblie comparativement à son activité endogène dans la *Corynebacterium spp.* de type sauvage, où la GntK a la séquence d'acides aminés SEQ ID NO : 2, pour obtenir une culture cellulaire ; et
2) la récolte de la L-lysine à partir de la culture cellulaire ou du micro-organisme,
dans lequel l'activité GntK est affaiblie par délétion totale ou partielle, substitution partielle ou insertion :
- sur un gène chromosomique codant pour la gluconate kinase ou
- sur un élément régulateur pour un gène chromosomique codant pour la gluconate kinase.

2. Utilisation d'un micro-organisme appartenant à une *Corynebacterium spp.,* ayant l'activité d'une gluconate kinase (GntK) affaiblie comparativement à son activité endogène dans la *Corynebacterium spp.* de type sauvage, où la GntK a la séquence d'acides aminés SEQ ID NO : 2, pour la production de L-lysine, où l'activité GntK est affaiblie par délétion totale ou partielle, substitution partielle ou insertion :
- sur un gène chromosomique codant pour la gluconate kinase ou
- sur un élément régulateur pour un gène chromosomique codant pour la gluconate kinase.

3. Procédé selon la revendication 1 ou utilisation selon la revendication 2, où la gluconate kinase (GntK) comprend en outre une séquence d'acides aminés de SEQ ID NO : 1.

4. Procédé selon la revendication 1 ou utilisation selon la revendication 2, où le micro-organisme producteur de L-lysine est *Corynebacterium glutamicum* déposé sous le numéro d'accession No. KCCM 11085P.


<110> CJ CheilJedang Corporation
<120> MICROORGANISM WITH ENHANCED L-LYSINE PRODUCTIVITY AND METHOD FOR PRODUCING L-LYSINE USING THE SAME
<130> OPA11076/PCT
<150> KR10-2010-0068618
   <151> 2010-07-15
<150> KR10-2011-0065694
   <151> 2011-07-01
<160> 12
<170> KopatentIn 1.71
<210> 1
   <211> 167
   <212> PRT
   <213> Corynebacterium glutamicum ATCC 13032
<220>
   <221> PEPTIDE
   <222> (1)..(167)
   <223> NCgl2399 gluconate kinase
<400> 1
   Met Ser Ala Ala Glu Gly Leu His Ile Val Val Met Gly Val Ser Gly
   1               5                   10                  15
   Cys Gly Lys Ser Ser Val Gly Lys Ala Leu Ala Ala Glu Leu Gly Ile
               20                  25                  30
   Glu Tyr Lys Asp Gly Asp Glu Leu His Pro Gln Glu Asn Ile Asp Lys
           35                  40                  45
   Met Ala Ser Gly Gln Ala Leu Asp Asp Asp Asp Arg Ala Trp Trp Leu
       50                  55                  60
   Val Gln Val Gly Lys Trp Leu Arg Asp Arg Pro Ser Gly Val Ile Ala
   65                  70                  75                  80
   Cys Ser Ala Leu Lys Arg Ser Tyr Arg Asp Leu Leu Arg Thr Lys Cys
                   85                  90                  95
   Pro Gly Thr Val Phe Val His Leu His Gly Asp Tyr Asp Leu Leu Leu
               100                 105                 110
   Ser Arg Met Lys Ala Arg Glu Asp His Phe Met Pro Ser Thr Leu Leu
           115                 120                 125
   Asp Ser Gln Phe Ala Thr Leu Glu Pro Leu Glu Asp Asp Glu Asp Gly
       130                 135                 140
   Lys Val Phe Asp Val Ala His Thr Ile Ser Glu Leu Ala Ala Gln Ser
   145                 150                 155                 160
   Ala Glu Trp Val Arg Asn Lys
                   165
<210> 2
   <211> 494
   <212> PRT
   <213> Corynebacterium glutamicum ATCC 13032
<220>
   <221> PEPTIDE
   <222> (1)..(494)
   <223> NCgl2905 gluconate kinase
<400> 2
   Met Gly Ser Ile Pro Thr Met Ser Ile Pro Phe Asp Asp Ser Arg Gly
    1               5                  10                  15
   Pro Tyr Val Leu Ala Met Asp Ile Gly Ser Thr Ala Ser Arg Gly Gly
               20                  25                  30
   Leu Tyr Asp Ala Ser Gly Cys Pro Ile Lys Gly Thr Lys Gln Arg Glu
           35                  40                  45
   Ser His Glu Phe Thr Thr Gly Glu Gly Val Ser Thr Ile Asp Ala Asp
       50                  55                  60
   Gln Val Val Ser Glu Ile Thr Ser Val Ile Asn Gly Ile Leu Asn Ala
   65                  70                  75                  80
   Ala Asp His His Asn Ile Lys Asp Gln Ile Ala Ala Val Ala Leu Asp
                   85                  90                  95
   Ser Phe Ala Ser Ser Leu Ile Leu Val Asp Gly Glu Gly Asn Ala Leu
               100                 105                 110
   Thr Pro Cys Ile Thr Tyr Ala Asp Ser Arg Ser Ala Gln Tyr Val Glu
           115                 120                 125
   Gln Leu Arg Ala Glu Ile Asp Glu Glu Ala Tyr His Gly Arg Thr Gly
       130                 135                 140
   Val Cys Leu His Thr Ser Tyr His Pro Ser Arg Leu Leu Trp Leu Lys
   145                 150                 155                 160
   Thr Glu Phe Glu Glu Glu Phe Asn Lys Ala Lys Tyr Val Met Thr Ile
                   165                 170                 175
   Gly Glu Tyr Val Tyr Phe Lys Leu Ala Gly Ile Thr Gly Met Ala Thr
               180                 185                 190
   Ser Ile Ala Ala Trp Ser Gly Ile Leu Asp Ala His Thr Gly Glu Leu
           195                 200                 205
   Asp Leu Thr Ile Leu Glu His Ile Gly Val Asp Pro Ala Leu Phe Gly
       210                 215                 220
   Glu Ile Arg Asn Pro Asp Glu Pro Ala Thr Asp Ala Lys Val Val Asp
   225                 230                 235                 240
   Lys Lys Trp Lys His Leu Glu Glu Ile Pro Trp Phe His Ala Ile Pro
                   245                 250                 255
   Asp Gly Trp Pro Ser Asn Ile Gly Pro Gly Ala Val Asp Ser Lys Thr
               260                 265                 270
   Val Ala Val Ala Ala Ala Thr Ser Gly Ala Met Arg Val Ile Leu Pro
           275                 280                 285
   Ser Val Pro Glu Gln Ile Pro Ser Gly Leu Trp Cys Tyr Arg Val Ser
           290                 295                 300
   Arg Asp Gln Cys Ile Val Gly Gly Ala Leu Asn Asp Val Gly Arg Ala
   305                 310                 315                 320
   Val Thr Trp Leu Glu Arg Thr Ile Ile Lys Pro Glu Asn Leu Asp Glu
                   325                 330                 335
   Val Leu Ile Arg Glu Pro Leu Glu Gly Thr Pro Ala Val Leu Pro Phe
               340                 345                 350
   Phe Ser Gly Glu Arg Ser Ile Gly Trp Ala Ala Ser Ala Gln Ala Thr
           355                 360                 365
   Ile Thr Asn Ile Gln Glu Gln Thr Gly Pro Glu His Leu Trp Arg Gly
       370                 375                 380
   Val Phe Glu Ala Leu Ala Leu Ser Tyr Gln Arg Val Trp Glu His Met
   385                 390                 395                 400
   Gly Lys Ala Gly Ala Ala Pro Glu Arg Val Ile Ala Ser Gly Arg Val
                   405                 410                 415
   Ser Thr Asp His Pro Glu Phe Leu Ala Met Leu Ser Asp Ala Leu Asp
               420                 425                 430
   Thr Pro Val Ile Pro Leu Glu Met Lys Arg Ala Thr Leu Arg Gly Thr
           435                 440                 445
   Ala Leu Ile Val Leu Glu Gln Leu Glu Pro Gly Gly Thr Arg Ala Thr
       450                 455                 460
   Pro Pro Phe Gly Thr Thr His Gln Pro Arg Phe Ala His His Tyr Ser
   465                 470                 475                 480
   Lys Ala Arg Glu Leu Phe Asp Ala Leu Tyr Leu Lys Leu Val
                   485                 490
<210> 3
   <211> 504
   <212> DNA
   <213> Corynebacterium glutamicum ATCC 13032
<220>
   <221> gene
   <222> (1)..(504)
   <223> nucleotide sequence of NCgl2399 gluconate kinase
<400> 3
   atgtcagcag ccgaaggctt acatattgtc gtcatgggcg tttctggctg cggcaaatcc     60
   tccgtcggta aagccctagc agcggagctc ggaatcgaat acaaagacgg cgacgaactt    120
   cacccccagg aaaacatcga caagatggcc tccggccagg cacttgacga cgacgaccgt    180
   gcatggtggc tagtccaggt tggcaagtgg ctccgcgacc gaccaagcgg cgtcatcgca    240
   tgctccgccc tcaagcgctc ctaccgcgat ctcctgcgca ccaaatgccc aggaaccgtc    300
   ttcgtccacc tccacggcga ctacgatctc ctactttccc gcatgaaggc ccgcgaagat    360
   cacttcatgc catccacctt gctagattcc caatttgcaa ccctcgagcc gctcgaagat    420
   gacgaagatg gcaaggtttt cgacgttgcc cacaccatca gcgaactggc cgcccaatct    480
   gcagagtggg ttcgcaacaa ataa                                           504
<210> 4
   <211> 1485
   <212> DNA
   <213> Corynebacterium glutamicum ATCC 13032
<220>
   <221> gene
   <222> (1)..(1485)
   <223> nucleotide sequence of NCgl2905 gluconate kinase
<400> 4 aaggcaagag agcttttcga cgccctctac ctcaagttgg tctag 1485
<210> 5
   <211> 26
   <212> DNA
   <213> Artificial Sequence
<220>
   <223> primer 2399F1
<400> 5
   gctctagacc ccagaacatg ctgacg 26
<210> 6
   <211> 26
   <212> DNA
   <213> Artificial Sequence
<220>
   <223> primer 2399R1
<400> 6
   ggggtacccg ctgctagggc tttacc 26
<210> 7
   <211> 27
   <212> DNA
   <213> Artificial Sequence
<220>
   <223> primer 2399F2
<400> 7
   ggggtaccgg aaccgtcttc gtccacc 27
<210> 8
   <211> 26
   <212> DNA
   <213> Artificial Sequence
<220>
   <223> primer 2399R2
<400> 8
   gctctagagt caccgctggt acatcc 26
<210> 9
   <211> 26
   <212> DNA
   <213> Artificial Sequence
<220>
   <223> primer 2905F1
<400> 9
   gctctagaca ttggagcatc cgtagc 26
<210> 10
   <211> 27
   <212> DNA
   <213> Artificial Sequence
<220>
   <223> primer 2905R1
<400> 10
   tcccccgggg ccttcaccat cgaccaa 27
<210> 11
   <211> 27
   <212> DNA
   <213> Artificial Sequence
<220>
   <223> primer 2905F2
<400> 11
   tcccccgggg ttcccgaaca gatcccc 27
<210> 12
   <211> 26
   <212> DNA
   <213> Artificial Sequence
<220>
   <223> primer 2905R2
<400> 12
   gctctagacg ctctgacctg cctaac 26